(19) Europäisches Patentamt
European Patent Office
Office européen des brevets

(11) **EP 4 012 721 A1**

(12) **EUROPEAN PATENT APPLICATION**

(43) Date of publication:
**15.06.2022 Bulletin 2022/24**

(51) International Patent Classification (IPC):
*G16H 50/30* *(2018.01)*

(21) Application number: **21153398.9**

(22) Date of filing: **26.01.2021**

(52) Cooperative Patent Classification (CPC):
**G16H 50/30**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **08.12.2020 PCT/CN2020/134472**

(71) Applicant: **Koninklijke Philips N.V.**
**5656 AG Eindhoven (NL)**

(72) Inventors:
• **JIANG, Ze Yu**
**5656 AE Eindhoven (NL)**
• **LIN, Qizhong**
**5656 AE Eindhoven (NL)**
• **TIAN, Cong**
**5656 AE Eindhoven (NL)**

(74) Representative: **Philips Intellectual Property & Standards**
**High Tech Campus 52**
**5656 AG Eindhoven (NL)**

(54) **HEMODYNAMIC INSTABILITY INDEX INDICATOR**

(57)    According to a concept of the invention, there is proposed a method and system for monitoring a subject. Subject data for the subject is received, the subject data including vital sign measurements and laboratory results. A VII is calculated from the received vital sign measurements, and a LII is calculated from the received laboratory results. The VII and the LII are combined according to a non-linear model so as to generate an indicator representing hemodynamic instability.

FIG. 6

EP 4 012 721 A1

**Description**

FIELD OF THE INVENTION

The invention relates to clinical decision support. It finds particular application in conjunction with predicting physiological and clinical status changes and will be described with particular reference thereto.

BACKGROUND OF THE INVENTION

When caring for subjects, the sooner a clinician learns of deterioration of a subject, the sooner the clinician can take corrective action. This, in turn, reduces the likelihood of end organ damage, especially in the case of hemodynamic deterioration, and generally improves subject outcome. Detecting deterioration typically requires a clinician to manually review physiological data for a plurality of physiological parameters, such as systolic blood pressure, and/or laboratory data. However, clinicians care for a large number of subjects and the ratio of subjects to clinicians is only expected to increase. Further, the frequency with which the physiological data is generated is high. As such, clinicians are often delayed in detecting deterioration.

To alleviate this, automatic monitoring of subjects is becoming increasingly prevalent. International Patent Application Publication Number WO2013171620 discloses a method of using a vital signs instability index (VII), laboratory instability index (LII) and looking up a prevalence value in a VII-to-prevalence table using the VII and the LII (the prevalence value indicating a prevalence of unstable VIX segments in a training population for differing VIX range and LIX bands). The VIX ranges span the range of possible values for the VII in predetermined increments, the LII bands represent differing degrees of risk with the physiological condition and include the range of possible values for the LII, wherein the VII segments of the training population correspond to VII trends over a predetermined period of time. The prevalence value is mapped to a value of indicator by linearly adjusting the prevalence value so it scales over the range of prevalence values in the VII-to-prevalence table.

However, the first challenges of this method is that a VII-to-prevalence table has to be pre-defined and great efforts are spent.

The second principal challenge with this automatic monitoring method is how to find the vital sign measurements or laboratory results that are most possibly lead to the deterioration of the subject and notify the clinicians. These key findings can provide timely support to the clinicians so that the clinicians can take suitable actions to alleviate the deterioration aiming directly at the key factors that lead to the deterioration.

SUMMARY OF THE INVENTION

The invention is defined by the claims.

In accordance with one aspect, a medical system for monitoring a subject is provided. The system comprises one or more processors programmed to: receive subject data for the subject, the subject data including vital sign measurements and laboratory results; calculate a vital signs instability index (VII) regarding a physiological condition of the subject from the received vital sign measurements; calculate a laboratory instability index (LII) regarding the physiological condition from the received laboratory results; and integrate the VII and the LII into an indicator of subject deterioration. The integration comprises: combining the VII and the LII according to a non-linear model to generate the indicator representing hemodynamic instability.

It is proposed to combine the VII and LII so as to generate an indicator (e.g. score) for hemodynamic instability. In particular, it is proposed to combine the VII and the LII according to a non-linear model so as to cater for model training with logistic regression.

For instance, where ground truth information is not available for both VII and LII, only have the ground truth information for hemodynamic instability may be available. However, training of a VII and a LII model may not be done with hemodynamic instability label information, because re-using the information of ground truth is not allowed. Accordingly, proposed embodiments may address the issue of having no VII and LII label information.

In particular, embodiments may add a sigmoid function to merge VII and LII values. In this way, the VII and the LII may be combined according to a non-linear model, and the model structure may similar to a multilayer perceptron (MLP) (but not a typical MLP fully connect neural network, because it combines two single-layer neural networks for example). An extra sigmoid function may be added as an activation function to convert linear combinations to non-linear combinations (because they describe the input-output relations in a non-linear way and give the model power to be more flexible in describing arbitrary relations).

Embodiments may provide an indicator (e.g. score) for hemodynamic instability. Such embodiments may therefore be of particular use for supporting clinical decision making. Exemplary usage applications may for example, relate to predicting the onset, treatment (outcome) or development of medical conditions and/or medical procedures. Embodiments may thus be of particular use in relation to cardiovascular assessment, for example.

In particular, embodiments may be used in relation to a subject (e.g. a subject) so as assist and/or optimize medical assessment, therapy and/or treatment for the subject. Such embodiments may support improved clinical assessment and/or planning. Improved Clinical Decision Support (CDS) may therefore be provided by proposed concepts.

In an embodiment, the non-linear model may comprise a non-fully-connected multilayer perceptron, MLP, that is trained to make a decision about the hemodynamic instability based on VII and LII.

Further, the non-fully connected MLP may comprise: a first neural network adapted to receive the VII as an input and output a VII value based on the VII; a second neural network adapted to receive the LII as an input and output a LII value based on the LII; and a non-linear function configured to merge VII and LII values.

The first neural network may comprise at least one sub neural network, each of the at least one sub neural network being adapted to receive one VII calculated based on vital sign measurements obtained by one device and output a VII value correspondingly. Also, the second neural network may comprise at least one sub neural network, each of the at least one sub neural network being adapted to receive one LII calculated based on laboratory results obtained in one examination category and output a LII value correspondingly. The non-linear function may then be configured to merge the at least one VII value and the at least one LII value.

In some embodiments, the non-fully-connected MLP may be trained using a backpropagation algorithm configured to send an error signal back toward a hidden layer of the MLP and to adjust the error signal using weights of the MLP.

By way of example, the physiological condition may be hemodynamic stability.

In some embodiments, the VII value may be calculated using a model modeling the relationship between instability of the physiological condition and the laboratory tests corresponding to the received vital sign measurements. And the LII value may be calculated using a model modeling the relationship between instability of the physiological condition and the laboratory tests corresponding to the received laboratory results.

In some embodiments, the at least one processor may be further programmed to: display the indicator using one or more of text, color coding, and a bar chart, the color codes identifying different degrees of risk of subject deterioration.

In some embodiments, the at least one processor may be further programmed to: display top ranking factors of each VII and LII with the top ranking weight values.

According to examples in accordance with another aspect of the invention, there is provided a method for monitoring a subject is provided. The method comprises: receiving subject data for the subject, the subject data including vital sign measurements and laboratory results; calculating a vital signs instability index, VII, regarding a physiological condition of the subject from the received vital sign measurements; calculating a laboratory instability index, LII, regarding the physiological condition from the received laboratory results; and integrating the VII and the LII into an indicator of subject deterioration. The integration of the VII and the LII comprises: combining the VII and the LII according to a non-linear model to generate the indicator representing hemodynamic instability.

According to examples in accordance with an aspect of the invention, there is provided a computer program comprising code means for implementing the method of any preceding claim when said program is run on a processing system.

In accordance with another aspect, a graphical user interface (GUI) for monitoring a subject is provided. The GUI includes a display of a deterioration indicator for the subject. The deterioration indicator integrates a vital signs instability index (VII) and a laboratory instability index (LII). The VII and LII indicate instability of a physiological condition of the subject. The VII is calculated from vital sign measurement, and the LII is calculated from laboratory results.

One potential advantage of the proposed concept(s) resides in an instability index taking into account multiple physiological parameters. Another advantage resides in reducing alerts. Another advantage resides in focusing a clinician's attention on subjects requiring extra vigilance. Another advantage resides in processing low-latency data separately from high- latency data. Another advantage resides in increased sensitivity to abnormal subject conditions. Another advantage resides in adaptability to available data. Another advantage resides in reducing the likelihood of outlying values triggering alerts. Another advantage resides in adjusting to cases in which a subject has conditions that are not typical of the average population.

Still further advantages of the present invention will be appreciated to those of ordinary skill in the art upon reading and understand the following detailed description.

These and other aspects of the invention will be apparent from and elucidated with reference to the embodiments described hereinafter.

BRIEF DESCRIPTION OF THE DRAWINGS

For a better understanding of the invention, and to show more clearly how it may be carried into effect, reference will now be made, by way of example only, to the accompanying drawings, in which:

Fig. 1 is a block diagram of an information technology (IT) infrastructure of a patient care environment;

Fig. 2 is a block diagram of a clinical decision support system;

Fig. 3 is a graphical representation of a vital signs instability index (VII) for hemodynamic stability and the corresponding inputs;

Fig. 4 is a graphical representation of a proposed model structure for combining a VII and LII to generate an indicator according to an embodiment;

Fig. 5 is a graphical representation of a baseline HII and a vital signs instability index;

Fig. 6 is a graphical representation of an HII for hemodynamic stability and the corresponding inputs;

Fig. 7 depicts a back propagation process for the proposed model structure of Fig. 6; and

Fig. 8 is a flow chart illustrating operation of an alternative embodiment of the CDDS of Fig. 1.

## DETAILED DESCRIPTION OF THE EMBODIMENTS

The invention will be described with reference to the Figures.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figures are merely schematic and are not drawn to scale. It should also be understood that the same reference numerals are used throughout the Figures to indicate the same or similar parts.

The invention provides concepts for determining a hemodynamic instability index (HII) from: (i) a vital sign instability index (VII) obtained from vital sign measurements; and (ii) a laboratory instability index (LII) from laboratory results. Such concepts may employ a non-linear model, and thus avoid a need for an extra reference (e.g. a prevalence table) to map and linearly adjust values.

In particular, in combining LII and VII to a final index (HII), each variable may have multiple parameters which affect them and, thus, if variables are provided with multiple parameters, the model will be a non-linear model. The entire structure may thus be a non-fully connection MLP model which considers correlation among different parameters. A non-fully connection MLP model may facilitate faster training (when compared to a fully connection MLP), and the non-fully connection MLP may allow combination of a linear index with non-linear model. In particular, a non-fully connection MLP may support fast propagation of a loss/error which, in turn, enables the algorithm to converge more quickly.

According to a concept of the invention, there is proposed a method and system for monitoring a subject. Subject data for the subject is received, the subject data including vital sign measurements and laboratory results. A VII is calculated from the received vital sign measurements, and a LII is calculated from the received laboratory results. The VII and the LII are combined according to a non-linear model so as to generate an indicator representing hemodynamic instability.

Illustrative embodiments may, for example, be employed in medical monitoring platforms as a clinical decision support system.

Implementations in accordance with the present disclosure relate to various systems, adaptions, and/or methods for indicating hemodynamic instability of a subject. According to proposed concepts, a number of possible solutions may be implemented separately or jointly. That is, although these possible solutions may be described below separately, two or more of these possible solutions may be implemented in one combination or another.

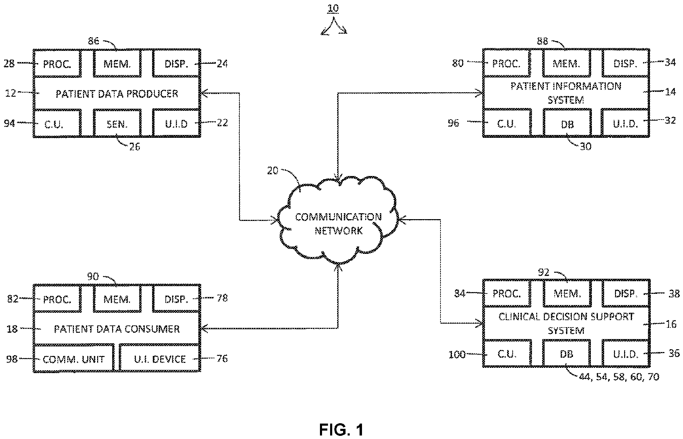

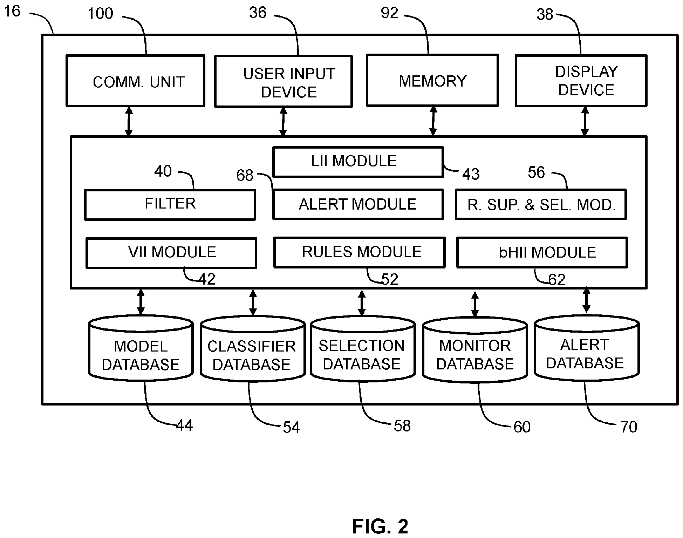

With reference to Figs. 1 and 2, an information technology (IT) infrastructure 10 of a subject monitored environment, such as an intensive care unit (ICU), caring for one or more subjects is provided. The IT infrastructure 10 includes one or more subject data producers 12, optionally a subject information system 14, a clinical decision support system (CDSS) 16, one or more subject data consumers 18, and the like. Suitably, the components of the IT infrastructure 10 are interconnected via a communication network 20, such as the Internet, a local area network, a wide area network, a wireless network, a virtual private network, or the like.

The subject data producers 12 generate subject data for corresponding subjects cared for in the subject monitored environment. The subject data for a subject includes one or more of physiological data, admission, discharge and transfer (ADT) data, laboratory data, clinical data, outcome data, and alert data. Physiological data includes one or more of physiological parameter waveforms, measurements of physiological parameters typically generated at a predetermined sample rate, such as 1 second or upon a change in the parameter, observed physiological parameters, and calculated physiological parameters. Examples of such physiological parameters include systolic blood pressure (SBP), heart rate (HR), Sp02, and so on. ADT data includes values for demographic data, such as age, nationality, and so on, and care preferences, such as do not resuscitate (DNR), comfort measures only (CMO), allow natural death (AND), reason for admission to the hospital or care unit, and so on. ADT data is typically generated when a subject is admitted to and/or discharged from a medical institution and/or transferred between subject care environments, such as the ICU and/or general ward, of a medical institution. Laboratory data includes laboratory test results and is typically generated sporadically upon the happening of an event, such as a clinician ordering a lab test. Clinical data includes data indicating actions taken to improve the health of the subject and other observations such as level of consciousness, intervention measures, dialysis, medications administered, the subject social and prior medical history, history of current illness, other social or risk factors the subject possesses, such as genomics, and so on. Outcome data includes data indicating the outcome of a subject's medical treatments and/or stay in the medical institution, such as whether the subject's condition worsened or improved, whether the subject died, and so on. Typically, the outcome data is generated during the subject's stay at a medical institution after medical interventions are taken. Alert data is data indicating an alert, such as deterioration of a subject, and is typically generated in response to detection of deterioration.

The subject data can be generated manually and/or automatically, typically depending upon the type of subject data. As to the former, user input 22 can be employed. Optionally, the subject data producers 12 include display devices 24 providing users with a user interface within which to manually enter the subject data and/or for displaying subject data to clinicians. As to the latter, sensors 26, such as Sp02 sensors, measuring, for example, physiological parameters and/or lab results and/or processors 28 monitoring and/or processing data, such as subject data, can be employed. Examples of subject data producers include, but are not limited to, physiologic monitors, mobile communications devices, laboratory systems, point of care systems, clinical information systems, and so on.

The subject information system 14 stores subject data from the IT infrastructure 10, such as from the subject data producers 12 and/or the CDSS 16, in one or more databases 30 of the IT infrastructure 10. For example, the subject information system 14 can store SBP for a subject from one of the subject data producers 12. The subject information system 14 is configurable to store subject data from user input devices 32 in the databases 30 and/or to allow stored subject data to be viewed on display devices 34. The display devices 34 can also be used to facilitate receipt of data from the user input devices 32. Suitably, the subject information system 14 manually stores subject data for a predetermined amount of time, such as a year, to allow other components of the IT infrastructures 10, such as the CDSS 16, to access historical subject data. Examples of subject information systems include, but are not limited to, electronic medical record systems, departmental systems, and the like.

The CDSS 16 receives subject data for the subjects from the IT infrastructure 10, such as from the subject data producers 12 and/or the subject information system 14. The CDSS can be alternatively configured so that subject data is also received from other sources, such as user input devices 36, optionally with display devices 38 providing users a user interface within which to enter the subject data, and/or sources, such as databases, external to the IT infrastructure 10. The subject data includes, for example, current subject data (e.g., current measurements of physiological parameters) and/or historical subject data (e.g., prior measurements of physiological parameters). Using the received subject data, the CDSS 16 monitors the wellbeing of the subjects. Monitoring includes, for example, generating alerts when the condition of a subject appears to be deteriorating, reports summarizing the state of the subjects, vital signs instability index (VII) values, and so on.

The CDSS 16 optionally also includes a filter 40. At least some of the received subject data passes through the filter 40 that conditions subject data into a standardized format and/or filters subject data that is not suited to monitoring the wellbeing of the subjects. Advantageously, conditioning subject data allows the CDSS 16 to be utilized in a variety of hosts and consume data from a variety of hosts in the native format. The filtering can include one or more of comparing the subject data to predetermined ranges of normalcy, ensuring the subject data meets time criteria for usability, and cross checking the subject data. For example, physiological data is typically filtered to remove measurements that aren't within predetermined boundaries, such as boundaries indicating possible values, and/or are otherwise highly unlikely. As another example, ADT data is typically filtered to remove ADT for subjects that do not belong to a targeted demographic (e.g., age, ethnicity, etc.). For example, ADT data for a subject which has not reached a predetermined age, such as the age of majority (e.g., generally 18 in the United States), and/or which exceeds a predetermined age, such as an improbable or unlikely age. As yet another example, laboratory results are typically filtered to remove results that aren't within predetermined boundaries, such as boundaries indicating possible values, and/or which exceed a predetermined age, such as 24 hours. Advantageously, this removes data which is stale and/or likely to be outlying thereby reducing the likelihood of false alerts.

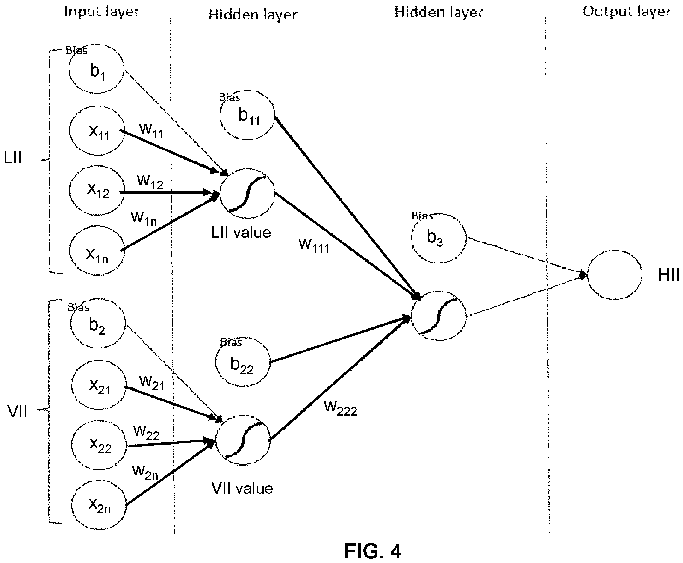

A VII module 42 of the CDSS 16 calculates VII values from the received subject data (as filtered, where relevant). That is, the VII module 42 is configured to generate a VII value based on the VII. The VII module 42 is based on a VII model (e.g. the second neural network in in Figure 4).

VII typically combines low-latency data, such as current physiological data, for a plurality of physiological parameters and, optionally, static data, such as demographic data, into a single measure reflective of stability of a physiological condition of a subject, such as the subject's hemodynamic status, pulmonary stability, nutritional stability, and so on. The CDSS 16 can be configured such that the VII values for the subjects are displayed on the display devices 38. The VII values can be calculated continuously and/or upon the happening of an event, such as a timer event, a user input event, the availability of new data, and so on. For example, a clinician can manually trigger calculation of a VII value for a subject so as to determine the hemodynamic stability of the subject. The CDSS 16 can further be configured such that the VII values are saved for historical analysis, typically in the subject information system 14.

A VII value for stability of a physiological condition is calculated by providing values for predictive variables to a selected VII model (e.g. the second neural network in Figure 4) that generates the VII value based on the predictive variables. The predictive variables are one or more of physiological parameters, features extracted from the static data, such as ethnicity, and the like relevant to determining the stability of the physiological condition. Suitably, the VII model is selected from a plurality of VII models in a model database 44 based on the physiological condition and/or the availability of data. For example, a first VII model is selected for stability of a first physiological condition and a second VII model is selected for stability of a second physiological condition. As another example, a first VII model is selected when measurements for HR and noninvasive SBP are available and a second VII model is selected when HR and invasive SBP are available. Further, the VII values produced by the models typically range between 0 and 1, where the closer the value is to 1, the more likely the subject is to be unstable. The VII models can be developed using any predictive model methodology, such as logistic regression, multinomial logistic regression, linear regression and support vector machine learning.

The generic VII models include for instance a logistic regression model for hemodynamic instability with the form of:

$$VII\ value = \frac{1}{1+e^{-z}} \qquad (1)$$

Where

$$z = \gamma + \beta_1 nSBP + \beta_2 nSI + \beta_3 Age \qquad (2)$$

Specific VII models are derived from different subject sub-populations (cardiogenic shock, hemorrhagic shock, septic shock and so on), and based on different source parameters (invasive SBP, non-invasive SBP, and the like). The model takes into account SBP and shock index (SI), which are highly significant predictive variables in determining hemodynamic stability, and returns a VII between zero and one. SI is the ratio of heart rated divided by SBP. The higher the VII, the less stable the subject. In some instances, $\beta$, the coefficient for SBP, is negative. As SBP gets lower, VII tends to increase, reflecting that the subject is approaching a less stable state. Further, $\beta 2$, the coefficient for SI, is positive. As SI gets higher, VII also tends to increase, again reflecting a decrease in stability. An approach for determining the coefficients is discussed hereafter.

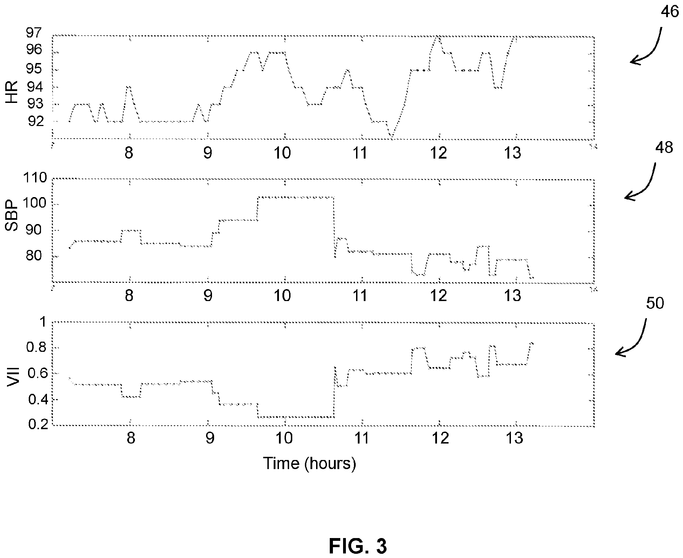

With reference to Fig. 3, a typical VII output for hemodynamic stability and the corresponding inputs as a function of time are illustrated. The inputs were drawn from real subject data. A first panel 46 and a second panel 48 of the plot show the HR and SBP, respectively, of a subject. As noted above, HR and SBP are highly significant predictive variables in determining hemodynamic stability. In a third panel 50, the calculated VII values are displayed. One can see that, by hour 11.75, the subject's VII value is rather high (nearly 0.8), indicating that the subject is not hemodynamically stable. Although not illustrated, at hour 14, the subject was administered a vasopressor, indicating that the hemodynamic instability was recognized by the clinician.

Referring back to Figs. 1 and 2, the VII module 42 can be further configured to generate and/or update the VII models from historical subject data. The historical subject data includes records for a plurality of subjects, where each records includes values for a plurality of variables, including the predictive variables, and corresponding outcome data indicating whether the subject was stable for the physiological conditions of the predictive models being updated and/or generated. Using the records, specifically the associations between the variables and the outcomes, and a predictive model methodology, such as logistic regression, multinomial logistic regression, linear regression and support vector machine learning, the predictive variables and/or one or more rules for predicting outcome are identified. For example, multivariable logistic regression can be employed to identify the predictive variables and the coefficients of the above described logistic regression model. When the VII module 42 is employed to update the VII models, the VII models are typically updated in response to an event, such as a periodic timer event, a user input event, the availability of new historical subject data, and so on.

VII may include two versions, nVII and iVII. The specific version of VII depends on whether it is calculated noninvasively or invasively. For example, when VII is used for hemodynamic stability, nVII can be VII determined using noninvasive BP and iVII can be VII determined using invasive BP. VII can be determined as described above in connection with the VII module 42 of Fig. 2.

LII values are suitably determined in the same manner as VII values, except that LII values are calculated from only lab data. A LII module 43 of the CDSS 16 calculates LII values from the received subject's lab data (as filtered, where relevant). That is, the LII module 43 is configured to generate an LII value based on the LII. The LII module 43 is based on an LII model (e.g. the first neural network in in Figure 4). Namely, a LII value is calculated by providing values for predictive variables to a selected LII model that generates the LII value based on the predictive variables. The predictive variables are one or more features extracted from lab data and which are predictive of instability on the physiological condition. LII value is typically based on logistic regression (e.g., a model of the same form as the model of Equation 1), but other models are contemplated.

Like VII, LII includes two versions, nLII and iLII. The version of LII used to determine the deterioration indicator depends upon the version of VII used to determine the deterioration indicator. Where nVII is used, nLII is used, and where iVII is used, iLII is used. For example, where LII represents hemodynamic instability, nLII is for a subject with noninvasive BP and iLII is for a subject with invasive BP. Both versions of LII use only lab values, but the lab values and the model parameters (e.g., logistic regression coefficients) that work best are somewhat different for subject episodes depending upon which version of LII is being used. For example, where LII represents hemodynamic instability and is used with a logistic regression model, the lab values and the weighting that work best vary depending upon whether BP is measured invasively or noninvasively. Continuing with this example, LII typically depends on albumin, BUN, and white blood cell count for noninvasively monitored subjects, and LII typically depends on the same set of labs with the addition of HC03 for invasively monitored subjects.

If a value for a lab parameter is missing that goes into the LII calculation, it is assumed that the subject has an average value for that lab parameter, the average value shared by a subject population to which the subject belongs. A value for a lab parameter could be missing, for example, because it has not been measured yet. Further, a sample-and-hold scheme is typical used with no time limitations for lab parameters used by the LII calculating. In a sample-and-hold scheme, a value for a lab parameter is typically used until replaced with a newer value. In some instances, varying schemes can be used depending upon the lab parameter. For example, a sample-and-hold scheme could only be used for HC03 so long as the most recent value is no older than a predetermined period of time, such as three hours. Otherwise, an average value shared by a subject population to which the subject belongs can be used.

For most lab parameters, a sample-and-hold scheme can be used for a parameter so long as the current value is no older than a time between twelve (12) and thirty six (36) hours. Thus, there can be provide an nLII value and an iLII value at all times for a subject.

Both nLII and iLII values are divided into a plurality of bands, typically three bands: a low risk band; a medium risk band; and a high risk band. At any point in time, a subject's deterioration indicator is based on the subject's VII value and the LII band that the corresponding LII value falls into.

Conventionally, the deterioration indicator is determined by using a VII-to - prevalence table which has rows corresponding to VII values and columns corresponding to the plurality of bands for both nVII and iVII. For example, the VII-to- prevalence table can include 100 rows corresponding to 100 possible VII values in 0.01 increments and 6 columns corresponding to six LII bands, including a low, medium and high risk band for each of nLII and iLII.

However, according to one or more proposed concepts, such an extra reference (e.g. prevalence table) to map and linearly adjust values is not required.

A rules module 52 of the CDSS 16, determines HII based on VII value and LII value calculated by the VII module 42 and the LII module 43 respectively.

In particular, with reference to Figure 4, it is proposed to combine VII and LII with one non-linear model. Such a combination of VII and LII to generate the indicator (i.e. HII) may be represented using the following equations (3) and (4):

$$HII = \frac{1}{1+e^{-z}} \qquad (3)$$

$$z = aVII\ value + \delta LII\ value \qquad (4)$$

As illustrated by Fig. 4, when visualized by graphs, the above mathematical expressions provide a model structure that is similar with a MultiLayer Perceptron (MLP). However, it is not a typical MLP fully connect neural network. Instead, it combines two one-layer neural networks and one more sigmoid function is added as activation function to convert linear combinations to non-linear combinations (because they describe the input-output relations in a non-linear way and give the model power to be more flexible in describing arbitrary relations).

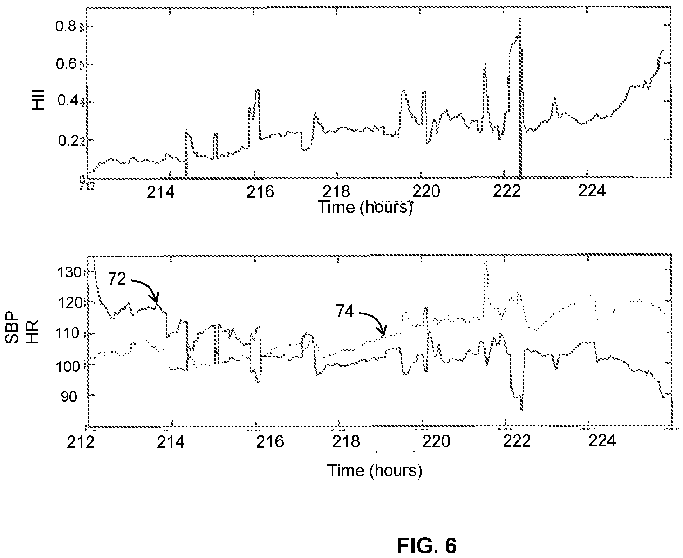

Specifically, Fig. 6 is a graphical illustration of a proposed non-linear model structure.

From Fig. 4, it can be seen that LII value may be represented by the following equation (5):

$$LII\ value = \frac{1}{1+e^{-(w_{11}x_{11}+w_{12}x_{12}+w_{13}x_{13}\cdots)}} \qquad (5)$$

$$VII\ value = \frac{1}{1+e^{-(w_{21}x_{21}+w_{22}x_{22}+w_{23}x_{23}\cdots)}} \qquad (6)$$

$$HII = \frac{1}{1+e^{-(w_{111}LII\ value+w_{222}VII\ value)}} \qquad (7)$$

Considering only Equation 5 an Equation 6, there are two linear models, respectively, since each variable only has one parameter to influence them. More specifically, LII value is a linear output of LII, VII value is a linear output of VII. But when LII value and VII value are combined to the final indicator HII (as represented by Equation 7 above), each variable has multiple parameters affecting affect them. For example, $x_{11}$ is not only influenced by $w_{11}$, it is also influenced by $w_{31}$. Therefore, because the variables are affected by multiple parameters, the model is non-linear.

The final indicator HII may have a value in the range [0, 1]. For example, if HII is close to one ('1'), the risk is high, and if HII is close to zero ('0'), the risk is low. Thus, based on the value of HII, a physician can decide the degree of patient deterioration.

The whole structure depicted in Fig. 4 is a non-fully connection MLP model, which is non-linear which considers correlation among different parameters. For this, the training processing is faster than a fully connection MLP, and the non-fully connection MLP allows the combination of a linear index with non-linear model.

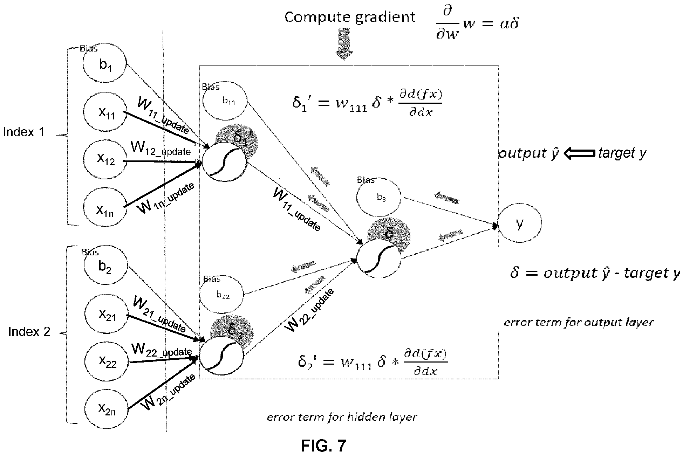

Typically, training a MLP model has three main steps, namely "forward pass"; "calculate loss or errors"; and "backward pass". For the step of "forward pass", the inputs are passed to the model. The inputs are then multiplied with weights and bias added at each layer. For the calculation of loss or errors calculation, generated (i.e. predicted) outputs from the mode are compared with real data or expected outputs. Based upon the comparison, the loss or errors can be calculated. After calculating the loss or errors, the loss/error is back-propagated and the weights of the model are updated using gradient, which is the main step in the training of the model. This back propagation process is illustrated in Figure 7. In this process, weights will adjust according to the gradient flow in that direction, the formula being represented by the following equation:

$$W' = W + Learning\ Rate * (expected - predicted) * X \qquad (8)$$

When the loss value converges to a minimum, the optimal parameters for the model can be obtained in order to provide correct/accurate outputs.

As mentioned above, although the model is like a MLP, the network is not a fully-connect neural network. Rather, multiple single-layer networks are merged together and one more layer is added to generate final outputs. After multiple iterations, optimal parameters may be obtained.

As shown in Fig. 4, the non-fully connected MLP may comprise: a first neural network adapted to receive the VII as an input and output a VII value based on the VII, a second neural network adapted to receive the LII as an input and output a LII value based on the LII, and a non-linear function configured to merge VII and LII values.

It's understood that the first neural network may comprise at least one sub neural network, each of the at least one sub neural network being adapted to receive one VII calculated based on vital sign measurements obtained by one device and output a VII value correspondingly. Also, the second neural network may comprise at least one sub neural network, each of the at least one sub neural network being adapted to receive one LII calculated based on laboratory results obtained in one examination category and output a LII value correspondingly. The non-linear function may then be configured to merge the at least one VII value and the at least one LII value.

The proposed concepts of separating variables into different groups, more specifically, using lab testing variables as the first index (LII), and using vital sign variables measured by different types of equipment, e.g. monitoring device, breathing machine... etc.) as one or more indexes ('VII1'....'VII2'... 'VII3'... etc.) and may assist in identifying key factors in different groups faster and more directly. In some embodiments, different types of lab texting variables can also be separated into different groups and used as one or more indexes ('LII1'....'LII2'...'LII3'... etc.)).

The final indicator HII may indicate a risk of subject deterioration, with higher values indicating a higher risk of subject deterioration. The calculated indicator HII can be displayed to a clinician for example.

If the value of the indicator HII (i.e. risk indicator) indicates that there is a shock risk (purely by way of example), a clinician or medical professional may hope to find the factors that lead to this risk. Instead of ranking all weights of the variables ($x_{11}$, $x_{12}$... $x_{21}$,$x_{22}$...) together and finding the top ranking weight values, proposed embodiments may rank the weights in each group (LII, VIII, VII2...), respectively, and find the top ranking weights in each group. For example, $w_{11}$, $w_{13}$ are the top ranking two weights in group LII, and $w_{22}$, $w_{23}$ are the top ranking two weights in the group VII. Proposed embodiments can provide support to the medical professional to decide which factors are the most relevant factors (e.g.,

$x_{11}$, $x_{22}$) by selecting from the corresponding factors $x_{11}$, $x_{13}$, $x_{22}$, $x_{23}$, that may lead to the shock risk. This can help avoid that all the top ranking weights are from a single group (LII or VII) which are not usually the case.

Hence, a final indicator of subject deterioration (such as an indicator representing hemodynamic instability) by combining a VII (regarding a physiological condition of the subject from the received vital sign measurements) and a LII (regarding the physiological condition from the received laboratory results) according to a non-linear model.

The final indicator can be directly or indirectly displayed to a clinician. As to the former, for example, the final value can be displayed to a clinician using text, a bar chart, or the like. As to the latter, for example, the range of indicator values can be divided into bands corresponding to the risk of subject deterioration. For example, the range of indicator values can be divided into thirds corresponding to low, medium and high risk of subject deterioration. The final indicator can then be mapped to one of these bands and the band can be identified to a clinician. The band is typically identified to a clinician using colors. For example, drawing on the foregoing example, red can indicate a high risk of subject deterioration, yellow can indicate a medium risk of subject denervation and green and indicate low risk of subject deterioration.

A graphical user interface (GUI) can be employed to display generated deterioration indicator values for a plurality of beds to a clinician (e.g., at a nurse station). For example, the deterioration indicator can be displayed with one or more of a text value between 0 and 100 representing the value (the range of text value [0, 100] represents the final indicator HII with a value in the range [0, 1]), a color to indicate low, medium, or high risk, and a bar chart volume representing the value. Values of 0 to 33.33 can be displayed with a green background (i.e., low risk), values between 33.33 and 66.66 can be displayed with a yellow background (i.e., medium risk), and all values greater than 66.66 can be displayed with a red background (i.e., high-risk). Labels proximate the deterioration indicators identify the corresponding subject by, for example, one or more of the subject's name and a subject identifier (PID). Other information, such as gender, can also be displayed. As illustrated, the deterioration indicators are displayed for hemodynamic instability, but the GUI is amenable to deterioration indicators for other physiological conditions.

When a deterioration indicator is selected with, for example, a user input device, the GUI can display additional information regarding the selected deterioration indicator. This additional information can include a history of the subject's deterioration indicator over a predetermined period, such as three or six hours. The history can be displayed as a trend line of deterioration indicator values, for example. The trend line can be color coded based on the risk region of the value at that point in time. As noted above, values of 0 to 33.33 can be displayed with a green background (i.e., low risk), values between 33.33 and 66.66 can be displayed with a yellow background (i.e., medium risk), and all values greater than 66.66 can be displayed with a red background (i.e., high-risk).

The additional information displayed on the GUI may further include the top ranking factors in each index ('LII1', 'LII2,'... 'VII1', 'VII2'... etc.) with the top ranking weight values. In this way, a clinician's attention is focused on factors requiring extra vigilance and the clinician can take suitable actions to alleviate the deterioration aiming directly at the key factors that lead to the deterioration.

As a clinician selects a deterioration indicator value (e.g., by dragging a mouse cursor over the trend line), a tool-tip can be shown for the selected deterioration indicator value. The tool-tip displays one or more of physiological parameters, lab parameters, and other relevant data for the deterioration indicator value. Such relevant data can include, for example, one or more of blood pressure, BUN, hematocrit, and other important laboratory and vital sign values. Further, such relevant data can include when displayed values were measured. Any values that are out of range can be highlighted in red or yellow to draw a clinician's attention to important pathophysiological issues that may be present.

The current deterioration indicator value can also be displayed next to the historical display. For example, the current deterioration indicator value can be represented as a bar chart with the volume and color reflecting the degree of risk, and a number positioned over the bar chart.

The rules module 52 of the CDSS 16 is further configured to determine HII thresholds indicating instability for the subjects. An HII threshold for a subject is determined based upon the corresponding physiological condition and, optionally, contextual data, such as laboratory data and/or demographic data. Contextual data is data describing one or more of where a subject is in the care process, the subject's problem list, interventions, demographics, laboratory tests, and the like. Contextual data is not directly relevant to the HII, but provides an indication as to where the threshold should be set for a particular subject. For example, a subject with a creatinine value of 0.9 mg/dL may be considered stable with an HII value of .5, whereas a subject with a creatinine of 3.2 mg/dL may not. When no contextual data regarding a subject is available, a generic threshold is employed for the subject. Otherwise, a threshold based on the provided contextual data is typically employed. For example, if contextual data, such as blood lab results that indicate a low hematocrit or albumin level, are generated, the HII threshold can be adjusted to a lower value.

The HII thresholds are suitably determined from rules of one or more HII classifiers of an HII classifier database 54 which discriminate between stability and instability given values for a plurality of variables, including HII and, optionally, one or more variables of contextual data, such as laboratory tests. The rules module 52 can optionally include an HII classifier for each possible set of input variables. For example, the rules module 52 can include an HII classifier for an input comprised of only an HII value and an HII classifier for an input comprised of an HII value and a first contextual value, such as a lab result for a particular lab test. The HII classifiers can be generated and/or updated using any machine learning methodology, such as decision tree algorithms. Advantageously, any rule set for discriminating between stable and unstable subjects using decision tree analysis will have a background rule, indicating a threshold for HII in the absence of any contextual data. For example, if HII is greater than 0.6, the subject is unstable. In addition to this background rule, the rule set includes rules incorporating contextual data, such as laboratory, and increasing or decreasing the HII threshold depending upon the context. For example, if HII is greater than 0.33 and creatinine is greater than 1.6, the subject is unstable.

In some instances, instead of rules with individual conditions based on lab results, the various lab results used in the rules could be integrated into a single laboratory instability index (LII). Values of LII can then be used to set HII thresholds. In some embodiments, combinations of VII and LII can be used to set HII thresholds.

The rules module 52 may also generate and/or update the HII classifiers from historical subject data. The historical subject data includes records for a plurality of subjects, where each record includes values for input variables, including VII and, optionally LII, variables of contextual data, such as laboratory tests, having a bearing on the monitoring sensitivity for the subject, and outcome data indicating whether the subject was unstable. Using the records, specifically the associations between the input variables and the outcomes, and a machine learning algorithm, such as a decision tree algorithm, one or more rules for determining the outcome are determined. When the rules module 52 is employed to update the classifiers, the HII classifiers are typically updated in response to an event, such as a periodic timer event, a user input event, the availability of new historical subject data, and so on.

A rule supervisor and selector module 56 determines a set of one or more monitoring rules and/or one or more VII models, LII models or HII models to employ for each subject to be monitored. A monitoring rule takes as input values for one or more variables, such as a physiological parameter, and provides an indication as to whether a subject is deteriorating. To determine a set of one or more monitoring rules and/or one or more VII models, LII models or HII models, one or more selection rules of a selection rules database 58 are employed. The selection rules select one or more monitoring rules from a plurality of monitoring rules in a rules monitoring database 60 and/or select one or more VII models, LII models and HII models from the model database 44. The monitoring rules are suitably generated manually by, for example, a clinical expert and/or automatically using a machine learning algorithm.

The selection rules can be based upon one or more of available subject data, subject context, and/or the source of the subject data. Typically, however, the selection rules are based upon contextual data, such as laboratory data and/or demographic data. Where contextual data is available, the selection rules determine a set of monitoring rules and/or VII models, LII models or HII models tailored to the available contextual data. Where contextual data is unavailable, the selection rules return a generic set of monitoring rules and/or a generic VII model, LII model or HII model selection. In this way, the rule supervisor and selector module 56 is adaptive to available subject data. While selection rules based on contextual data are typical, other selection schemes are contemplated. For example, it is contemplated that the rule supervisor and module 56 simply returns a set of monitoring rules for every subject regardless of the availability of contextual data. Similar to the monitoring rules, the selection rules are suitably generated manually by, for example, a clinical expert and/or automatically using a machine learning algorithm.

The monitoring rules may be based on HII. However, one challenge with employing HII is that a high HII can be the result of outlying physiological data due to, for example, a misplaced monitoring lead or a subject's sudden change in arm position, or movement from a prone to a sitting or standing position. Especially where automatic subject monitoring is employed, such data can lead to false alerts. To reduce the likelihood of these false alerts, a baseline HII (bHII) indicating how the HII has been behaving is calculated by a bHII module 62 of the CDSS 16. The bHII module 62 calculates bHII values from historical subject data. Many methods can be used to estimate the trend in a series of HII values. Some are more sophisticated than others. In one example, the bHII value is the maximum VII value or the 90 percentile value within the past predetermined amount of time, such as three hours.

When a bHII is employed, an alert exceeding a trigger threshold is indicated only if a bHII indicates a rising trend in HII. Then the current high HII value is more likely to reflect the true state of the physiological condition. Otherwise a false alert is more likely. In one example, a rising trend is detected when the current HII value is more than a predetermined threshold and bHII is at least some fraction of the threshold (e.g., 3/4 or 2/3). This means the current HII is high enough to cause alert and an earlier HII is already quite high. Then and only then is an alert raised. If bHII is too low, then the current HII, though high, is more likely to be an aberration and no alert is raised. This specific implementation has the advantage of being simple and efficient and has proved to be effective in reducing alerts due to data outliers.

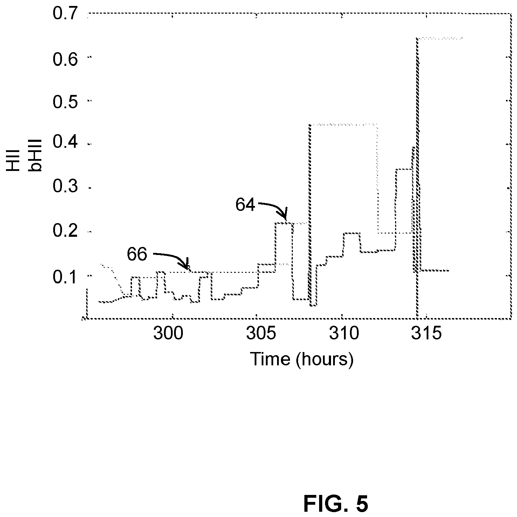

With reference to Fig. 5, an example of HII 64 and the corresponding bHII 66 calculated for a subject during a portion of their stay in the subject care environment as a function of time since admission is provided. With reference to around hour 308, the subject's HII reaches a high enough value to trigger an alert. However, because the bHII at this point in time (-0.22) is too low, no alert is triggered as a result of this spike in HII. In this way, the implementation of bHII has made it impossible to trigger an alert due to this quite possibly aberrational data. Later in the subject's stay, around hour 314.5, the subject's HII again becomes high enough to cross a threshold for instability. This time, however, the bHII is also high enough (~ 0.4) to allow an alert to be triggered. Essentially, bHII is sensitive to trends in data; a high HII resulting from a gradual increase in HII values is far more likely a result of actual important physiological changes than is a HII that suddenly increases.

Referring back to Figs. 1 and 2, an alert supervisor module 68 monitors the subject data for the subjects and generates alerts when deterioration is detected. To determine when a subject is deteriorating, the alert supervisor module 68 employs the set of monitoring rules for the subject determined by the rule supervisor and selector module 56. As noted above, the monitoring rules take as input values for one or more variables, such as physiological parameters, typically received from the IT infrastructure 10. Further, the input variables can include HII calculated based on VII and LII. The HII are received from, for example, the rules module 52 and generated, for example, according to the HII model selections made by the rule supervisor and selector module 56. Further, the thresholds for the monitoring rules for the HII are received from the rules module 52.

When it is determined that a subject is deteriorating, an alert to that affect is generated. The alert is suitably generated and addressed to a clinician according to one or more rules in an alert rules database 70. The rules can take into account one or more of hospital policy, clinician worklists, on-call status of clinicians, clinician preferences, and so on. For example, suppose hospital policy specifies that in response to an alert of a particular type, alert the on-call physician overseeing the physician. Further, suppose the on-call physician wishes to be contacted in a particular way, such as text message. The rules can be employed to generate and send an alert meeting these requirements. Alert escalation is also contemplated. Alert escalation is the idea of escalating an alert by resending it to the same or a different clinician after conditions, such as the passing of a predetermined amount of time without receiving an acknowledgment, are met.

In addition to sending the alert, alerts for the same deterioration are disarmed (i.e., prohibited from being triggered) until a rearming condition is met. The rearming conditions can include, for example, the passage of a predetermined amount of time. Further, the rearming conditions can, for example, be determined by an adaptive rearming method. The adaptive method presupposes familiarity with the typical intervention measures taken by clinicians in response to deterioration of the physiological condition corresponding to the disarmed alert and the availability of clinical data indicating interventions taken by clinicians. Further, the adaptive method presupposes an index or parameter that reflects a subject's stability with regard to the physiological condition. For example, when the physiological condition is hemodynamic stability, the index or parameter can be HII and typical intervention measures include the administration of fluids, vasopressors, or packed red blood cells.

According to the adaptive method, if an alert for deterioration of a physiological condition is issued, alerts for the same deterioration are prohibited from being issued for a predetermined amount of time, such as three hours. The predetermined amount of time corresponds to the lead time of the prediction that deterioration will occur and typically varies depending on the physiological condition. After the predetermined amount of time has passed, a determination is made as to whether intervention measures have been taken to address the deterioration based on clinical data typically received from the IT infrastructure 10 and knowledge of typical intervention measures for the physiological condition.

If no intervention has been or is being administered, alerts for the same deterioration are disarmed unless and/or until the index or parameter has worsened by a threshold amount compared to the index or parameter value at the time of the initial alert. The threshold amount can be fixed or variable, such as half of the distance to a previous index or parameter value. By rearming in this way, the lack of intervention by a clinician after a significant time has passed is interpreted as an indication that the subject's condition at the time of the first alert is acceptable for that particular subject. Therefore, even though the index or parameter is abnormal, or unstable, by population standards, the adaptive method learns that it is normal. If intervention is being administered, this is recognized as acknowledgement by clinicians and further alerts are unnecessary. After the intervention has ended, alerts for deterioration of the physiological condition are rearmed after a predetermined amount of time passes.

With reference to Fig. 6, a plot of the HII for a subject and the corresponding inputs, SBP 72 and HR 74, over the course of several hours is illustrated. An alert is generated at hour 214.5 as the HII of the subject crosses the alert threshold. During the course of the three hours that follows the first alert, no intervention is taken. This is interpreted to mean that the subject's dynamics at the time of the first alert are acceptable for that particular subject. Therefore, after three hours have passed, no alert is generated because the subject's HII is not much higher than it was at the time of the first alert. By the time hour 222.5 is reached, however, HII has significantly increased and, therefore another alert is issued for the subject. It should be noted that at hour 226, the clinician administered vasopressor indicating that, indeed, the subject experienced a clinically notable incident of hemodynamic instability.

Referring back to Figs. 1 and 2, the subject data consumers 18 consume subject data from the IT infrastructure 10, such as from the subject data producers 12, the CDSS 16, the subject information system 14, and so on, for the subjects. For example, the subject data consumers 18 can receive HII from the CDSS 16. As another example, the subject data consumers 18 can receive respiration rate and heart rate from the subject data producers 12. As yet another example, the subject data consumers 18 can receive alerts from the CDSS 16. Optionally, the subject data consumers 18 also receive subject data from user input devices 76, optionally with display devices 78 providing users a user interface within which to enter the subject data. Examples of subject data consumers include, but are not limited to, subject monitors, spot check subject monitors, mobile communications devices, subject information systems, clinical decision support systems, and so on.

Consumption can include processing the received subject data to generate additional subject data and/or consolidating the subject data into reports. A report is a computer file in a format, such as PDF, DOCX, DOC, and so on. Optionally, newly generated subject data and/or newly generated reports are saved in the IT infrastructure 10, such as in the subject information system 14. Further, optionally, newly generated reports are electronically messaged to clinicians using, for example, email and/or printed using, for example, a laser printer, an inkjet printer, and so on. Consumption can also include displaying the received subject data, such as alerts or HII, for at least one subject on a user interface presented to clinicians via the display devices 78. The user interface is typically continuously updated as subject data is received. Advantageously, this allows clinicians to monitor subjects in near real time.

When displaying subject data and/or generating a report, the report and/or display suitably includes at least subject name and HII for at least one subject. Where the received subject data includes subject data for a plurality of subjects, the received subject data is suitably formatted in a table structure with a plurality of rows corresponding to the subjects. The rows can optionally be sorted and/or can be sorted by severity of HII. For example, a clinician can employ the user input devices 76 to sort a table of subject data based on HII. Further, clinicians can optionally selectively view the details of a HII. For example, a clinician can employ the user input devices 76 to select a HII for a subject and view the variables and respective values that yielded the HII, optionally ranked based on contribution. Even more, the subject data can optionally be grouped based on similar HIIs. Groups include, for example, one or more of very low risk, low risk, moderate risk, high risk, and so on.

A HII can be represented as one or more of textual values (e.g., scores, probabilities, and so on), icons (e.g., one or more of shape, color, background, and so on based on severity), a combination of the foregoing, and so on in a user interface and/or a report. For example, a HII can be represented as a circle having a background color dependent upon severity, such as red for high risk, yellow for medium risk, and green for low risk. An icon can further includes a textual value overlaid thereon, optionally depending upon severity. For example, an icon can include a probability overlaid thereon when the severity is medium. A HII can also be presented to the use as a discrete message (e.g., alert, text page, email, SMS, and so on) or as a parameter based on the absolute probability that the subject will have an instability event in a preconfigured or continuous time horizons, or as a normalized scale of overall prediction of instability based on HII and other CDSS algorithms running for the subject.

The components of the IT infrastructure 10 suitably include processors 28, 80, 82, 84 executing computer executable instructions embodying the foregoing functionality, where the computer executable instructions are stored on memories 86, 88, 90, 92 associated with the processors 28, 80, 82, 84. The processor(s) 84 of the CDSS 16, for example, execute computer instructions on the one or more memories 92 of the CDSS 16 embodying the functionality of one or more of the filter 40, the VII module 42, alert module 68, the rules module 52, the rule supervisor and selector module 56 and the bHII module 62. It is, however, contemplated that at least some of the foregoing functionality can be implemented in hardware without the use of processors. For example, analog circuitry can be employed. Further, the components of the IT infrastructure 10 include communication units 94, 96, 98, 100 providing the processors 28, 80, 82, 84 an interface from which to communicate over the communication network 20. Even more, although the foregoing components of the IT infrastructure 10 were discretely described, it is to be appreciated that the components can be combined. For example, the subject data consumers 12 and the subject data producers 18 can be the same and/or have overlap. As another example, the CDSS 16 can be integrated with the subject data consumers 18 and/or the subject data producers 12. As yet another example, the CDSS 16, the subject data consumers 18 and the subject data producers 12 can be combined into a standalone device independent from the communication network 20.

We will now consider an exemplary operation of a CDSS according to one embodiment thereof.

The CDSS receives subject data from a physiological data feed, an ADT data feed, a laboratory data feed, and a clinical data feed.

The physiological feed provides measurements for HR, SBP (invasive and/or noninvasive), mean blood pressure (MBP) (invasive and/or noninvasive), and heart rhythm status; the ADT data feed provides a subject's age, whether a subject does not want to be resuscitated, whether a subject wants comfort measures only, and whether a subject wants to allow a natural death; the laboratory data feed provides lab results for blood tests, including tests for creatinine (Cr), blood urea nitrogen (BUN), albumin (Alb), hematocrit (HCT), hemoglobin (HB), white blood cell (WBC) count, bicarbonate (HC03), and prothrombin time (PT); and the clinical data feed describes medications (Meds) provided to a subject, whether the subject is administered dialysis, whether a subject has a intra-aortic balloon pump (IABP), any other drugs and the like administered to a subject and any samples drawn from a subject. Typically, the data feeds are the subject data producers 12.

At least some of the subject data, including physiological data, ADT data and laboratory data, passes through a filter. The filter standardizes the format of the data and/or ensures the HR and SBP are within possible ranges, e.g., noninvasive MBP is less than noninvasive SBP, and the noninvasive MBP drops 50% of the associated noninvasive SBP drop. Further, the filter ensures the subject age is reasonable and exceeds a predetermined age, such as the age of majority (e.g., generally 18 in the United States). Even more, the filter ensures Cr, BUN, Alb, HCT, HB, WBC, HC03 and PT are within possible ranges and the tests were performed within 24 hours (i.e., the time to live (TTL) of laboratory tests is only 24 hours).

The filtered subject data passes to a rule supervisor and selector module where it is used by the rule supervisor and selector module to determine a set of one or more monitoring rules and/or one or more VII models to employ for each subject to be monitored. The available VII models include a noninvasive model and an invasive model. The subject data is used to provide a context for the subject and based on this context, the appropriate monitoring rules and VII model(s) are selected. The determination of VII models passes to a VII module, which calculates VII values from the filtered subject data (VII) using the selected VII model(s). The VII values calculated by the VII module pass to a bHII module, which calculates bHII values from the VII values using a three hour moving window. The determined set of monitoring rules passes to the rules module 52, which determines thresholds for the VII values.

An alert supervisor module receives the rules and thresholds, as well as the HII and bVII values, and monitors for subject deterioration through application of the rule set. Upon determining deterioration, the alert supervisor notifies users of subject deterioration and disarms alerts for the same deterioration. Alerts are rearmed after 12 hours pass or 3 hours if HII worsens. Further, the monitoring rules do not alert if the subject is DNR, CMO, or AND and/or CR and BUN rules are suppressed if the subject is on dialysis. The alert supervisor module also has the ability to receive feedback from the user via the CDSS regarding the user's desired alert behavior. In the case, the CDSS is deployed where there is no connection to interventions charted in the subject information system, the user has the ability to indicate that an intervention is planned, thus suppressing new alerts until the user indicates the intervention is complete or the condition worsens to a new threshold that is based on the first alert and type of intervention indicated by the user.

The foregoing dealt with the generation of alerts indicating subject deterioration based on HII. However, one challenge with alerts is that alerts aren't always user friendly. Alerts can prove to be an annoyance to clinicians caring for subjects and can provide clinicians with an information overload. To alleviate these challenges with alerts, an indicator indicating subject deterioration can be used in lieu of alerts.

The functionality regarding the deterioration indicator, described above, can be implemented within the components 12, 14, 16, 18, typically the CDSS 16, of the IT infrastructure 10 of Fig. 1.

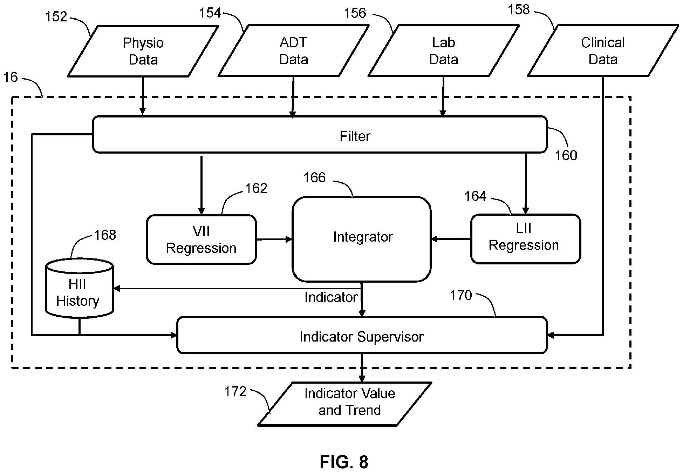

With reference to Fig. 8, a flow chart illustrating operation of an alternative embodiment of the CDSS 16 of Fig. 1 is illustrated. In contrast with the embodiment described in connection with Fig. 1, this embodiment pertains to the determination and use of the deterioration indicator described above. The components of the CDSS 16 of Fig. 1 are as described above, except that the processors 84 are reprogrammed.

According to the flowchart, the CDSS 16 receives subject data from a physiological data feed 152, an ADT data feed 154, a laboratory data feed 156, and a clinical data feed 158. The physiological feed 152 provides measurements for HR, heart rhythm status, BP (invasive and/or noninvasive), and mode (i.e., whether a noninvasive or invasive mode is being used); the ADT data feed 154 provides a subject's age, whether a subject does not want to be resuscitated, whether a subject wants comfort measures only, and whether a subject wants to allow a natural death; the laboratory data feed 156 provides lab results for blood tests, including tests for Cr, BUN, Alb, HCT, HB, WBC, HC03, and PT; and the clinical data feed 158 describes medications (Meds) provided to a subject, whether the subject is administered dialysis, whether a subject has IABP, any other drugs and the like administered to a subject and any samples drawn from a subject. Typically, the data feeds 152, 154, 156, 158 are the subject data producers 12, shown in Fig. 1.

Typically, at least some of the subject data, including physiological data, ADT data and laboratory data, passes through a filter 160. The filter 160 standardizes the format of the data and/or ensures the parameters of the data being filtered are within plausible ranges. For example, the filter 160 ensures the subject age is reasonable and exceeds a predetermined age, such as the age of majority (e.g., generally 18 in the United States). Typically, the filter 160 is the same as, or otherwise includes, the filter 40 of FIGURE 2. A VII module 162 and a LII module 164 generate VII values (i.e., iVII and/or nVII) and LII values (i.e., iLII and/or nLII), respectively, from the received subject data, optionally as filtered. The VII and LII values are determined as described above in connection with the generation of the deterioration indicator HII. Typically, the VII module 162 is the same as, or otherwise includes, the VII module 42 of Fig. 2. Further, the LII module 164 is the same as, or otherwise includes, the LII module 43 of Fig. 2. Typically, the VII module 162 and/or the LII module 164 use logistic regression models. However, other models can be employed.

Although not shown, it is contemplated that the models used by the VII module 162 and/or the LII module 164 can be selected by a rule supervisor and selector module. The rule supervisor and selector module uses the received subject data, optionally as filtered, to determine one or more VII models and/or one or more LII models to employ for the subject to be monitored. Typically, the available models include noninvasive and invasive variants for both LII and VII, since the models vary based on whether invasive or noninvasive. The rule supervisor and selector module uses the received subject data to provide a context for the subject and based on this context, the appropriate models are selected. The rule supervisor and selector module is typically a variant of the rule supervisor and selector module 56 of Fig. 2, the rule supervisor and selector module 56 of Fig. 2 extended for LII model selection.

An information integrator module 166 receives LII values and VII values from the LII and VII modules 164, 162 and generates deterioration indicator values (e.g. hemodynamic instability index (HII) values) as described above. As to the latter, the LII and VII are combined using a non-fully connection MLP model (as described above with reference to FIGURE 4 for example).

Typically, before generating deterioration indicator values, the information integrator module 166 filters the HII using HII baseline values to remove outliers, as described above. The HII baseline values are typically determined as described above using an HII history database 168, which stores HII values generated by the integrator module 166. The HII baseline values typically include upper bHII values and lower bHII values. As described above, a upper bHII value is the maximum HII value, or a percentile, such as 90 percentile, over a previous period of time (e.g., the three preceding hours). Further, a lower bHII value is the minimum HII value, or a percentile, such as 10 percentile, over a previous period of time (e.g., the three preceding hours). After determining a deterioration indictor value, the deterioration indicator value can be displayed, as described above.

For example, a deterioration indicator value can be directly or indirectly displayed to a clinician, for example, using the subject data consumers 18 of Fig. 1. As to the former, for example, the final value can be displayed to a clinician using text, a bar chart, or the like. As to the latter, for example, the range of indicator values can be divided into bands corresponding to the risk of subject deterioration. The final indicator can then be mapped to one of these bands and the band can be identified to a clinician. The band is typically identified to a clinician using colors.

An optional indicator supervisor 170 receives the deterioration indicator values and corresponding HII data from the information integrator 166 and the HII history database 168, respectively. Using the received HII data, the indicator supervisor 170 can monitor HII trends and mark HII for possible alerting using the re-arming method, described above. As noted above, the re-arming method is controlled by max HII, marking HII that are higher than max HII (by some threshold) and re-setting max HII to the current value of HII. Further, max HII can be re-set to the current HII if the current HII drops to a specified fraction of max HII.

When marked HII exceed an alert threshold, the corresponding deterioration indicator, when displayed, can be highlighted. The deterioration indicator can be displayed as described above, for example, using a GUI as described above. A display 172 of deterioration indicator values and corresponding trends is used with color coding. Additionally, or alternatively, when marked HII exceed an alert threshold, alerts can be generated to, for example, notify clinicians.

At least one of the processors 28, 80, 82, 84 of Fig. 1, typically the processors 84 of the CDSS 16, execute processor executable instructions embodying the foregoing functionality, including the functionality of the filter 160, the VII module 162, the LII module 164, the information integrator 166, and the indicator supervisor 170. The processor executable instructions are suitably embodied by corresponding memories 86, 88, 90, 92 of the processors 28, 80, 82, 84. At least one of the memories 86, 88, 90, 92, typically the memories 92 of the CDSS 16, include the VII history database 168.

As used herein, a memory includes one or more of a non-transient computer readable medium; a magnetic disk or other magnetic storage medium; an optical disk or other optical storage medium; a random access memory (RAM), read-only memory (ROM), or other electronic memory device or chip or set of operatively interconnected chips; an Internet/Intranet server from which the stored instructions may be retrieved via the Internet/Intranet or a local area network; or so forth. Further, as used herein, a processor includes one or more of a microprocessor, a microcontroller, a graphic processing unit (GPU), an application-specific integrated circuit (ASIC), a field-programmable gate array (FPGA), and the like; a user input device includes one or more of a mouse, a keyboard, a touch screen display, one or more buttons, one or more switches, one or more toggles, and the like; a display device includes one or more of a liquid crystal display (LCD), an light emitting diode (LED) display, a plasma display, a projection display, a touch screen display, and the like; and databases include one or more memories.

The invention has been described with reference to the preferred embodiments. Modifications and alterations may occur to others upon reading and understanding the preceding detailed description. For example, while VII was discussed as a means of detecting unstable subjects, VII could also be used to determine which subjects are in a safely stable state in order to prioritize his/her time or to decide who can be moved from, for example, the ICU to the general ward, since a prolonged, very low VII value is indicative of a very stable subject. It is intended that the invention be construed as including all such modifications and alterations insofar as they come within the scope of the appended claims or the equivalents thereof.

It will also be understood that the disclosed methods are computer-implemented methods. As such, there is also proposed a concept of a computer program comprising code means for implementing any described method when said program is run on a processing system.

The skilled person would be readily capable of developing a processor for carrying out any herein described method. Thus, each step of a flow chart may represent a different action performed by a processor, and may be performed by a respective module of the processing processor.

As discussed above, a proposed system may make use of a processor to perform data processing. The processor can be implemented in numerous ways, with software and/or hardware, to perform the various functions required. The processor typically employs one or more microprocessors that may be programmed using software (e.g. microcode) to perform the required functions. The processor may be implemented as a combination of dedicated hardware to perform some functions and one or more programmed microprocessors and associated circuitry to perform other functions. Examples of circuitry that may be employed in various embodiments of the present disclosure include, but are not limited to, conventional microprocessors, application specific integrated circuits (ASICs), and field-programmable gate arrays (FPGAs).

In various implementations, the processor may be associated with one or more storage media such as volatile and non-volatile computer memory such as RAM, PROM, EPROM, and EEPROM. The storage media may be encoded with one or more programs that, when executed on one or more processors and/or controllers, perform the required functions. Various storage media may be fixed within a processor or controller or may be transportable, such that the one or more programs stored thereon can be loaded into a processor.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. A computer program may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted that the term "adapted to" is intended to be equivalent to the term "configured to". Any reference signs in the claims should not be construed as limiting the scope.

**Claims**

1.  A medical system for monitoring a subject, said system comprising: one or more processors programmed to:

    receive subject data for the subject, the subject data including vital sign measurements and laboratory results;
    calculate a vital signs instability index, VII, regarding a physiological condition of the subject from the received vital sign measurements;
    calculate a laboratory instability index, LII, regarding the physiological condition from the received laboratory results; and
    integrate the VII and the LII into an indicator of subject deterioration,
    wherein the integration comprises:
    combining the VII and the LII according to a non-linear model to generate the indicator representing hemodynamic instability.

2.  The system of claim 1, wherein the non-linear model comprises a non-fully-connected multilayer perceptron, MLP, that is trained to make a decision about the hemodynamic instability based on VII and LII.

3.  The system of claim 2, wherein non-fully connected MLP comprises:
    a first neural network adapted to receive the VII as an input and output a VII value based on the VII;

    a second neural network adapted to receive the LII as an input and output a LII value based on the LII; and
    a non-linear function configured to merge VII and LII values.

4.  The system of claim 3, wherein:

    the first neural network comprises at least one sub neural network, each of the at least one sub neural network being adapted to receive one VII calculated based on vital sign measurements obtained by one device and output a VII value correspondingly;
    the second neural network comprises at least one sub neural network, each of the at least one sub neural network being adapted to receive one LII calculated based on laboratory results obtained in one examination category and output a LII value correspondingly; and
    the non-linear function is configured to merge the at least one VII value and the at least one LII value.

5.  The system of claim 2, 3 or 4, wherein the non-fully-connected MLP is trained using a backpropagation algorithm configured to send an error signal back toward a hidden layer of the MLP and to adjust the error signal using weights of the MLP.

6. The system of any of claims 1 to 5, wherein the physiological condition is hemodynamic stability.

7. The system of any of claims 1 to 6, wherein
   the VII value is calculated using a model modeling the relationship between instability of the physiological condition and the laboratory tests corresponding to the received vital sign measurements; and
   the LII value is calculated using a model modeling the relationship between instability of the physiological condition and the laboratory tests corresponding to the received laboratory results.

8. The system of any of claims 1 to 7, wherein the at least one processor (84) is further programmed to:
   display the indicator using one or more of text, color coding, and a bar chart, the color codes identifying different degrees of risk of subject deterioration.

9. The system of any of claims 8, wherein the at least one processor (84) is further programmed to:
   display top ranking factors of each VII and LII with the top ranking weight values.

10. A method for monitoring a subject, said method comprising:

    receiving subject data for the subject, the subject data including vital sign measurements and laboratory results;
    calculating a vital signs instability index, VII, regarding a physiological condition of the subject from the received vital sign measurements;
    calculating a laboratory instability index, LII, regarding the physiological condition from the received laboratory results; and
    integrating the VII and the LII into an indicator of subject deterioration,
    wherein the integration comprises:
    combining the VII and the LII according to a non-linear model to generate the indicator representing hemodynamic instability.

11. The method of claim 10, wherein the non-linear model comprises a non-fully-connected multilayer perceptron, MLP, that is trained to make a decision about the hemodynamic instability based on VII and LII.

12. The method of claim 11, wherein non-fully connected MLP comprises:

    a first neural network adapted to receive the VII as an input and output a VII value based on the VII;
    a second neural network adapted to receive the LII as an input and output a LII value based on the LII; and
    a non-linear function configured to merge VII and LII values.

13. The method of claim 12, wherein:

    the first neural network comprises at least one sub neural network, each of the at least one sub neural network being adapted to receive one VII calculated based on vital sign measurements obtained by one device and output a VII value correspondingly;
    the second neural network comprises at least one sub neural network, each of the at least one sub neural network being adapted to receive one LII calculated based on laboratory results obtained in one examination category and output a LII value correspondingly; and
    the non-linear function is configured to merge the at least one VII value and the at least one LII value.

14. The method of any of claims 10 to 13, wherein
    the VII value is calculated using a model modeling the relationship between instability of the physiological condition and the laboratory tests corresponding to the received vital sign measurements; and
    the LII value is calculated using a model modeling the relationship between instability of the physiological condition and the laboratory tests corresponding to the received laboratory results.

15. A computer program product comprising computer program code means which, when executed on a computing device having a processing system, cause the processing system to perform all of the steps of the method according to any of claims 10 to 14.

FIG. 1

16

100 ─╮     36 ─╮      92 ─╮           38 ─╮

| COMM. UNIT | USER INPUT DEVICE | MEMORY | DISPLAY DEVICE |
|---|---|---|---|

LII MODULE ─ 43

40 ─╮     68 ─╮                              56 ─╮

| FILTER | ALERT MODULE | R. SUP. & SEL. MOD. |
|---|---|---|

| VII MODULE | RULES MODULE | bHII MODULE |
|---|---|---|

─42              ─52              ─62

| MODEL DATABASE | CLASSIFIER DATABASE | SELECTION DATABASE | MONITOR DATABASE | ALERT DATABASE |
|---|---|---|---|---|

─44        ─54        ─58        ─60        ─70

**FIG. 2**

**FIG. 3**

Input layer     Hidden layer     Hidden layer     Output layer

FIG. 4

FIG. 5

FIG. 6

FIG. 7

FIG. 8

| | Europäisches Patentamt European Patent Office Office européen des brevets | **EUROPEAN SEARCH REPORT** | Application Number EP 21 15 3398 |

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | KIM WON-YOUNG ET AL: "A risk scoring model based on vital signs and laboratory data predicting transfer to the intensive care unit of patients admitted to gastroenterology wards", JOURNAL OF CRITICAL CARE, GRUNE AND STRATTON, ORLANDO, FL, US, vol. 40, 19 April 2017 (2017-04-19), pages 213-217, XP085176094, ISSN: 0883-9441, DOI: 10.1016/J.JCRC.2017.04.024 * Abstract; paragraphs [0001], [02.2], [02.3], [0004] * | 1-15 | INV. G16H50/30 |
| Y | Fang Andrew ET AL: "Early warning score validation methodologies and performance metrics: a systematic review", BMC medical informatics and decision making, 18 June 2020 (2020-06-18), pages 111-111, XP55817032, London DOI: 10.1186/s12911-020-01144-8 Retrieved from the Internet: URL:https://bmcmedinformdecismak.biomedcentral.com/track/pdf/10.1186/s12911-020-01144-8.pdf [retrieved on 2021-06-23] * the whole document * | 1-15 | |

TECHNICAL FIELDS SEARCHED (IPC)

G16H

-/--

The present search report has been drawn up for all claims

| Place of search | Date of completion of the search | Examiner |
|---|---|---|
| Munich | 24 June 2021 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 21 15 3398

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y | CHURPEK MATTHEW M. ET AL: "Multicenter Comparison of Machine Learning Methods and Conventional Regression for Predicting Clinical Deterioration on the Wards :", CRITICAL CARE MEDICINE., vol. 44, no. 2, 1 February 2016 (2016-02-01), pages 368-374, XP55817496, US ISSN: 0090-3493, DOI: 10.1097/CCM.0000000000001571 Retrieved from the Internet: URL:https://www.ncbi.nlm.nih.gov/pmc/articles/PMC4736499/pdf/nihms753330.pdf> * the whole document * | 1-15 | |
| Y | JOON-MYOUNG KWON ET AL: "An Algorithm Based on Deep Learning for Predicting In-Hospital Cardiac Arrest", JOURNAL OF THE AMERICAN HEART ASSOCIATION, vol. 7, no. 13, 3 July 2018 (2018-07-03), pages 1-11, XP055660397, DOI: 10.1161/JAHA.118.008678 * the whole document * | 1-15 | |
| Y | WO 2013/171620 A1 (KONINKL PHILIPS NV [NL]) 21 November 2013 (2013-11-21) * claims; figures * | 1-15 | TECHNICAL FIELDS SEARCHED (IPC) |
| Y | WO 2017/141131 A1 (KONINKLIJKE PHILIPS NV [NL]) 24 August 2017 (2017-08-24) * claims; figures * | 1-15 | |
| Y | US 2020/160998 A1 (WARD KEVIN R [US] ET AL) 21 May 2020 (2020-05-21) * paragraphs [0045], [0052]; claims; figures * | 1-15 | |

The present search report has been drawn up for all claims

| Place of search | Date of completion of the search | Examiner |
|---|---|---|
| Munich | 24 June 2021 | Vogt, Titus |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

**ANNEX TO THE EUROPEAN SEARCH REPORT
ON EUROPEAN PATENT APPLICATION NO.**

EP 21 15 3398

This annex lists the patent family members relating to the patent documents cited in the above-mentioned European search report.
The members are as contained in the European Patent Office EDP file on
The European Patent Office is in no way liable for these particulars which are merely given for the purpose of information.

24-06-2021

| Patent document cited in search report | | Publication date | Patent family member(s) | | | Publication date |
|---|---|---|---|---|---|---|
| WO 2013171620 | A1 | 21-11-2013 | BR | 112014028599 | A2 | 27-06-2017 |
| | | | CN | 104487975 | A | 01-04-2015 |
| | | | EP | 2850550 | A1 | 25-03-2015 |
| | | | JP | 6298454 | B2 | 20-03-2018 |
| | | | JP | 2015519941 | A | 16-07-2015 |
| | | | RU | 2014151237 | A | 10-07-2016 |
| | | | US | 2015145691 | A1 | 28-05-2015 |
| | | | WO | 2013171620 | A1 | 21-11-2013 |
| WO 2017141131 | A1 | 24-08-2017 | CN | 108738299 | A | 02-11-2018 |
| | | | EP | 3416542 | A1 | 26-12-2018 |
| | | | JP | 2019509101 | A | 04-04-2019 |
| | | | RU | 2018133125 | A | 19-03-2020 |
| | | | US | 2019029533 | A1 | 31-01-2019 |
| | | | WO | 2017141131 | A1 | 24-08-2017 |
| US 2020160998 | A1 | 21-05-2020 | NONE | | | |

EPO FORM P0459

For more details about this annex : see Official Journal of the European Patent Office, No. 12/82

**EP 4 012 721 A1**

**Patent documents cited in the description**

- WO 2013171620 A